# EUROPÄISCHE PATENTANMELDUNG

(11) **EP 1 438 954 A1**
(43) Veröffentlichungstag der Anmeldung: **21.07.2004**
(21) Anmeldenummer: 03001070.6
(22) Anmeldetag: 18.01.2003
(51) Int. Cl.: A61K 7/48, A61P 17/00

(54) **Erhöhung der Thermostabilität und Verbesserung der Fliesseigenschaften von chitosanhaltigen Formulierungen**

(71) Anmelder: Beiersdorf AG, 20245 Hamburg (DE)
(72) Erfinder: Kröpke, Rainer, 22869 Schenefeld (DE); Lindemann, Wiebke, 20257 Hamburg (DE)

(57) **Zusammenfassung**

Die vorliegende Erfindung betrifft Chitosan enthaltende kosmetische und/oder dermatologische Formulierungen, deren Temperaturstabilität durch Zusatz von Cellulose und/oder ihren Derivaten entscheidend erhöht und deren Fließeigenschaften auf diese Weise erheblich verbessert werden.

## Beschreibung

Die vorliegende Erfindung betrifft Chitosan enthaltende kosmetische und/oder dermatologische Formulierungen, deren Temperaturstabilität durch Zusatz von Cellulose und/oder ihren Derivaten entscheidend erhöht und deren Fließeigenschaften auf diese Weise erheblich verbessert werden.

Chitosan, das teilweise deacetylierte und teilweise depolymerisierte Derivat des Chitins ist ein vielseitig einsetzbarer Bestandteil einer Reihe von Arzneimitteln und kosmetischen Formulierungen. Es entsteht bei der alkalischen Behandlung von Chitin.

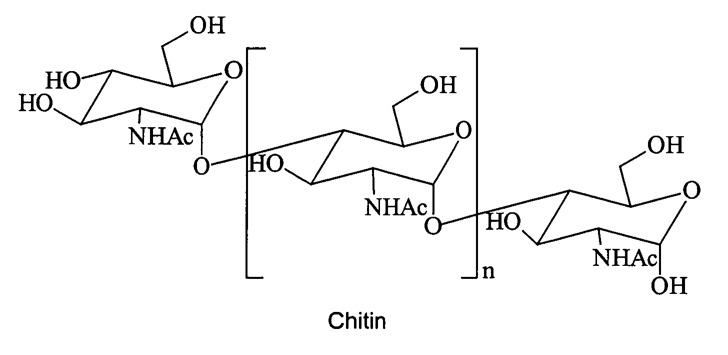

In der Pharmazie findet Chitosan Verwendung als Bindemittel in Tabletten und kann dazu dienen, Wirkstoffe mit Verzögerung abzugeben. In kosmetischen und/oder dermatologischen Zubereitungen fördert Chitosan die Wundheilung, da es die Löslichkeit von Wirkstoffen erhöht und darüber hinaus antimikrobiell (bakteriostatisch, fungistatisch) wirkt. Letzteres ist auch der Grund für den Einsatz von Chitosan als Konservierungsmittel in Kosmetika (EP 377.091). Es dient darüber hinaus als Feuchthaltemittel (sog.

Moisturizer), da es in der Lage ist Wasser zu binden. Chitosan lässt sich einsetzen zur Herstellung von Antitranspirantien, Badepräparaten (JP 6310.715) und Haarpflegemitteln (JP 63.277.607). Es dient als Träger für Riechstoffe in Seifen und Haarwaschmitteln (JP 6354.499) sowie, auf Cellulose aufgezogen, zur Herstellung gut haftbarer Make-up-Präparate (JP 0386.807).

Chitosan wird darüber hinaus als Stabilisator für stark saure Emulsionen sowie als Stabilisator und Verdickungsmittel in Polyvinylalkohol-Emulsionen eingesetzt. Ferner kann es zur Darstellung von Hydrogelen dienen. In Zubereitungen stabil ist Chitosan in Verbindung mit nichtionogenen, wasserlöslichen Gummen, Stärke, Glucose, Saccharose, Polyolen, Ölen, Fetten, Wachsen, nichtionogenen Emulgatoren und Säuren wie Essig-, Salz-, Salpeter-, Ameisen-, Citronen- und Milchsäure.

In Zubereitungen nicht stabil ist Chitosan in Verbindung mit ionischen Gummen, Alkalien, sulfonierten Netzmitteln, Schwefelsäure und ihren Salzen, den Sulfaten.

Kosmetische und/oder dermatologische Formulierungen, die Chitosan oder andere kationische Polymere enthalten, sind in der Regel nicht temperaturstabil. So zeigen sie im Lagertest ab 40 °C nach spätestens ein bis drei Monaten eine irreversible Wasserabscheidung. Die Aufgabe der vorliegenden Erfindung war es daher, dem Mangel des Standes der Technik abzuhelfen und kosmetischen und/oder dermatologischen Formulierungen, die Chitosan oder andere kationische Polymere enthalten, eine höhere Temperaturstabilität zu verschaffen.

Desweiteren bleiben bei Applikation kosmetisch und/oder dermatologischer Formulierungen aus Kunststoffverpackungen mit Schraubverschluss und konisch nach außen gewölbter Lochöffnung, wie sie für gewöhnlich für kosmetische Milche oder Lotions verwendet werden, nach Applikation des Kosmetikums in der Regel Reste desselben an der Außenseite der konisch nach außen gewölbten Lochöffnung haften. Diese werden vom Anwender für gewöhnlich durch Abwischen mit den Fingern entfernt. Durch diesen unhygienischen Vorgang steigt das Risiko einer Verkeimung der kosmetischen und/oder dermatologischen Formulierung im Verpackungsbehältnis. Es war deshalb die Aufgabe der vorliegenden Erfindung, die rheologischen Eigenschaften der kosmetischen und/oder dermatologischen Formulierung derart zu gestalten, das nach Applikation keine Reste an der Verpackungsöffnung zurückbleiben.

Es zeigte sich nun, und darin besteht die Lösung der Aufgabe, dass kosmetische und/oder dermatologische Formulierungen, die Chitosan oder andere kationische Polymere enthalten, überraschender Weise durch Zusatz von Cellulose und/oder ihren Derivaten deutlich an Temperaturstabilität gewinnen und ein stark verbessertes Applikationsverhalten aufweisen.

Vorteilhaft im Sinne der Erfindung erweisen sich dabei kosmetische und/oder dermatologische Formulierungen, die Chitosan in einer Konzentration von 0,05 bis 5 Gew.-% und insbesondere von 0,35 bis 2,25 Gew.-%, bezogen auf das Gesamtgewicht der Formulierung enthalten.

Desweiteren ist erfindungsgemäß vorteilhaft, in den kosmetischen und/oder dermatologischen Formulierungen Cellulose und/oder ihre Derivate in einer Konzentration von 0,01 bis 1,25 Gew.-%, insbesondere in einer Konzentration von 0,05 bis 0,75 Gew.-% bezogen auf das Gesamtgewicht der Formulierung einzusetzen.

Das erfindungsgemäß vorteilhafte Verhältnis von Chitosan zu Cellulose beträgt dabei 25 :1 bis 5:1 . Ganz besonders vorteilhaft ist das Verhältnis 20:1 bis 10:1 .

Es werden als Cellulosederivate erfindungsgemäß vorteilhaft eingesetzt : Celluloseacetat, Cellulosefettsäureester, Celluloseether, Carboxymetylcellulose, Ethylcellulose, Hydroxypropylcellulose, Methylcellulose, Cellulosemethylether, Methylethylcellulose und ganz besonders vorteilhaft Hydroxyethylcellulose.

Die kosmetischen und/oder dermatologischen Formulierungen sind bei Lagerung in einem Temperaturbereich von -15°C bis +50 °C mindestens 3 Monate stabil.

Die erfindungsgemäßen kosmetischen und/oder dermatologischen Formulierungen zeichnen sich ferner durch ihre besonderen Fließeigenschaften aus. Überschüssige Reste der Formulierung fließen nach Applikation aus einer Kunststoffflasche mit Schraubverschluss und konisch nach außen gewölbter Lochöffnung, wie sie gewöhnlich für kosmetische Milche, Lotionen und andere kosmetischer Formulierungen ähnlicher Konsistens verwendet werden, vollständig in das Verpackungsbehältnis wieder zurück, ohne an der konisch nach außen gewölbten Flaschenöffnung in Teilen haften zu bleiben. Dieser Vorgang verläuft mit erfindungsgemäßen Formulierungen mit einer Rückflussgeschwindigkeit R von über 0,4 cm /min, wobei die Rückflussgeschwindigkeit R definiert ist als die Länge des Füllgutstranges (der kosmetischen und/oder dermatologischen Formulierung) in cm, die unter dem Einfluss der Schwerkraft pro Minute in das aufrechtstehende Verpackungsbehältnis zurückfließt.

Die erfindungsgemäßen kosmetischen und/oder dermatologischen Formulierungen liegen vorteilhaft in Form eines Hydrodispersionsgels vor.

Des Weiteren können die erfindungsgemäßen kosmetischen und/oder dermatologischen Formulierungen vorteilhaft verwendet werden als Hautpflegeprodukte, sowie als "Aftersun"-Produkte. Dabei versteht man unter "Hautpflegeprodukte" im Sinne der Erfindung unter anderem Hautcremes, Hautlotionen, Milche, Salben, Öle, Balsame und Sera. "Aftersun-Produkte" sind dazu bestimmt, die Haut nach dem Sonnenbad zu kühlen und ihr Feuchthaltevermögen zu verbessern, wobei die Vermittlung des Kühleffektes eine zentrale Rolle spielt. Dieser Kühleffekt wird in der Regel durch hohe Mengen an Ethanol und Wasser erzielt, welches beim Verteilen der Formulierung auf der Haut spontan verdunstet. Ferner enthalten diese Präparate meist Feuchthaltemittel wie Glycerin oder Propylenglycol und entzündungshemmende Verbindungen wie zum Beispiel Allantion, α-Bisabolol, Panthenol oder Aloe-vera-Extrakt.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **O/W-Emulsionen** | | | | | |
|---|---|---|---|---|---|
| | **1** | **2** | **3** | **4** | **5** |
| Chitosan | 1,0 | 0,5 | 0,25 | 2,0 | 3,0 |
| Lecithin | 1,0 | 1,5 | 1,75 | 3,0 | 2,0 |
| Paraffinöl | 2,5 | 1,0 | 8,0 | 2,5 | 1,5 |
| Vaseline | 8,0 | 6,0 | 0,5 | 12,0 | 2,5 |
| Celluloseacetat | 0,05 | --- | 0,5 | --- | --- |
| Decyloleat | 0,5 | 0,75 | 1,0 | 2,0 | 0,25 |
| Octyldodecanol | 0,5 | 1,0 | 0,75 | 3,0 | 0,25 |
| Hydroxyethylcellulose | --- | 0,3 | --- | 0,05 | --- |
| Dicaprylylcarbonat | 0,1 | 0,05 | 0,15 | 0,5 | 1,0 |
| Methylcellulose | --- | --- | --- | --- | 1,0 |
| Glycerin | 3,0 | 5,0 | 10,0 | 15,0 | 1,5 |
| Milchsäure | 0,6 | 0,3 | 0,15 | 1,2 | 1,8 |
| Parfum | q,s, | q,s, | q,s, | q,s, | q,s, |
| Ethanol | 2,0 | --- | 15,0 | --- | 8,0 |
| Capryl-/Caprinsäuretriglycerid | 2,0 | 2,5 | 3,0 | 5,0 | 0,5 |
| Methyparaben | 0,4 | 0,15 | 0,05 | 0,3 | 0,4 |
| Propylparaben | 0,3 | 0,4 | 0,25 | 0,15 | --- |
| Wasser | ad 100 | ad 100 | ad 100 | ad 100 | ad 100 |

| **O/W-Emulsionen** | | | | | |
|---|---|---|---|---|---|
| | **6** | **7** | **8** | **9** | **10** |
| Chitosan | 1,0 | 0,5 | 0,25 | 2,0 | 3,0 |
| Lecithin | 1,0 | 1,5 | 1,75 | 3,0 | 2,0 |
| Paraffinöl | 2,5 | 1,0 | 8,0 | 2,5 | 1,5 |
| Methylcellulose | 0,25 | --- | 0,5 | --- | --- |
| Tocopherol | 0,5 | 0,75 | 1,0 | 2,0 | 0,25 |
| Octyldodecanol | 0,5 | 1,0 | 0,75 | 3,0 | 0,25 |
| Cellulosefettsäureester | --- | 0,3 | --- | --- | --- |
| Dicaprylylcarbonat | 0,1 | 0,05 | 0,15 | 0,5 | 1,0 |
| Cellulosemethylether | --- | --- | --- | 5,0 | 1,0 |
| Glycerin | 3,0 | 5,0 | 10,0 | 15,0 | 1,5 |
| Milchsäure | 0,6 | 0,3 | 0,15 | 1,2 | 1,8 |
| Parfum | q,s, | q,s, | q,s, | q,s, | q,s, |
| Ethanol | 3,0 | --- | 10,0 | --- | 20,0 |
| Capryl-/Caprinsäuretriglycerid | 2,0 | 2,5 | 3,0 | 5,0 | 0,5 |
| Methyparaben | 0,4 | 0,15 | 0,05 | 0,3 | 0,4 |
| Propylparaben | 0,3 | 0,4 | 0,25 | 0,15 | --- |
| Wasser | ad 100 | ad 100 | ad 100 | ad 100 | ad 100 |

| **O/W-Emulsionen** | | | | | |
|---|---|---|---|---|---|
| | **11** | **12** | **13** | **14** | **15** |
| Chitosan | 1,0 | 0,5 | 0,25 | 2,0 | 3,0 |
| Lecithin | 1,0 | 1,5 | 1,75 | 3,0 | 2,0 |
| Paraffinöl | 2,5 | 1,0 | 8,0 | 2,5 | 1,5 |
| Cocoglyceride | 8,0 | 6,0 | 0,5 | 12,0 | 2,5 |
| Celluloseacetat | 0,05 | --- | 0,5 | --- | --- |
| hydriertes Polylsobuten | 0,5 | 0,75 | 1,0 | 2,0 | 0,25 |
| Octyldodecanol | 0,5 | 1,0 | 0,75 | 3,0 | 0,25 |
| Hydroxyethylcellulose | --- | 0,3 | --- | 0,05 | --- |
| Dicaprylylcarbonat | 0,1 | 0,05 | 0,15 | 0,5 | 1,0 |
| Methylcellulose | --- | --- | --- | --- | 1,0 |
| Glycerin | 3,0 | 5,0 | 10,0 | 15,0 | 1,5 |
| Glycolsäure | 0,6 | 0,3 | 0,15 | 1,2 | 1,8 |
| Parfum | q,s, | q,s, | q,s, | q,s, | q,s, |
| Ethanol | 2,0 | --- | 15,0 | --- | 8,0 |
| Capryl-/Caprinsäuretriglycerid | 2,0 | 2,5 | 3,0 | 5,0 | 0,5 |
| Methyparaben | 0,4 | 0,15 | 0,05 | 0,3 | 0,4 |
| Propylparaben | 0,3 | 0,4 | 0,25 | 0,15 | --- |
| Wasser | ad 100 | ad 100 | ad 100 | ad 100 | ad 100 |

| **O/W-Emulsionen** | | | | | |
|---|---|---|---|---|---|
| | **16** | **17** | **18** | **19** | **20** |
| Chitosan | 1,0 | 0,5 | 0,25 | 2,0 | 3,0 |
| Lecithin | 1,0 | 1,5 | 1,75 | 3,0 | 2,0 |
| Paraffinöl | 2,5 | 1,0 | 8,0 | 2,5 | 1,5 |
| Methylcellulose | 0,25 | --- | 0,5 | --- | --- |
| Cycolmethicone | 0,5 | 0,75 | 1,0 | 2,0 | 0,25 |
| Octyldodecanol | 0,5 | 1,0 | 0,75 | 3,0 | 0,25 |
| Cellulosefettsäureester | --- | 0,3 | --- | --- | 0,05 |
| Dicaprylylcarbonat | 0,1 | 0,05 | 0,15 | 0,5 | 1,0 |
| Dimethicon | --- | --- | --- | 5,0 | 1,0 |
| Glycerin | 3,0 | 5,0 | 10,0 | 15,0 | 1,5 |
| Milchsäure | 0,6 | 0,3 | 0,15 | 1,2 | 1,8 |
| Parfum | q,s, | q,s, | q,s, | q,s, | q,s, |
| Ethanol | 3,0 | --- | 10,0 | --- | 20,0 |
| Capryl-/Caprinsäuretriglycerid | 2,0 | 2,5 | 3,0 | 5,0 | 0,5 |
| Methyparaben | 0,4 | 0,15 | 0,05 | 0,3 | 0,4 |
| Propylparaben | 0,3 | 0,4 | 0,25 | 0,15 | --- |
| modifizierte Stärke | --- | 2,5 | --- | 0,15 | --- |
| Wasser | ad 100 | ad 100 | ad 100 | ad 100 | ad 100 |

## Patentansprüche

1. Kosmetische und/oder dermatologische Formulierungen enthaltend
a) Chitosan und
b) Cellulose und/oder Cellulosederivate
neben anderen kosmetischen und dermatologischen Wirk- und Hilfsstoffen.

2. Kosmetische und/oder dermatologische Formulierungen nach Anspruch 1 enthaltend
a) Chitosan in einer Konzentration von 0.05 bis 5 Gew.-% und besonders bevorzugt in einer Konzentration von 0.35 bis 2.25 Gew.-%, bezogen auf das Gesamtgewicht der Formulierung
b) Cellulose und/oder Cellulosederivate in einer Konzentration von 0.01 bis 1.25 Gew.-% und bevorzugt in einer Konzentration von 0.05 bis 0.75 Gew.-%, bezogen auf das Gesamtgewicht der Formulierung.

3. Kosmetische und/oder dermatologische Formulierungen nach einem der Ansprüche 1 bis 2, **dadurch gekennzeichnet, dass** das Cellulosederivat/ die Cellulosederivate aus den Verbindungen Celluloseacetat, Cellulosefettsäureester, Celluloseether, Carboxymetylcellulose, Ethylcellulose, Hydroxypropylcellulose, Methylcellulose, Cellulosemethylether, Methylethylcellulose und/oder Hydroxyethylcellulose gewählt wird.

4. Kosmetische und/oder dermatologische Formulierungen nach einem der Ansprüche 1 bis 3, **dadurch gekennzeichnet dass** das Cellulosederivat Hydroxyethylcellulose ist.

5. Kosmetische und/oder dermatologische Formulierungen nach einem der Ansprüche 1 bis 4, **dadurch gekennzeichnet dass** sie bei Lagerung in einem Temperaturbereich von -15°C bis +50°C mindestens drei Monate stabil sind.

6. Kosmetische und/oder dermatologische Formulierungen nach einem der Ansprüche 1 bis 5, **dadurch gekennzeichnet dass** überschüssige Reste der Formulierung nach Applikation aus einer Kunststoffflasche mit Schraubverschluss und konisch nach außen gewölbter Lochöffnung, wie sie gewöhnlich für kosmetische Milche, Lotions und andere kosmetischer Formulierungen ähnlicher Konsistenz verwendet werden, vollständig in das Verpackungsbehältnis wieder zurückfließen, ohne an der konisch nach außen gewölbten Flaschenöffnung in Teilen haften zu bleiben.

7. Kosmetische und/oder dermatologische Formulierungen nach einem der Ansprüche 1 bis 6, **dadurch gekennzeichnet dass** die Rückflussgeschwindigkeit R größer als 0,4 cm/min ist.

8. Kosmetische und/oder dermatologische Formulierungen nach einem der Ansprüche 1 bis 7, **dadurch gekennzeichnet, dass** sie in Form eines Hydrodispersionsgels vorliegt.

9. Verwendung von kosmetischen und/oder dermatologischen Formulierungen nach einem der Ansprüche 1 bis 8 zur Verbesserung der Fließeigenschaften kosmetischer und/oder dermatologischer Zubereitungen.

10. Verwendung von kosmetischen und/oder dermatologischen Formulierungen nach einem der Ansprüche 1 bis 9 zur Erhöhung der Temperaturstabilität kosmetischer und/oder dermatologischer Zubereitungen.

11. Verwendung von kosmetischen und/oder dermatologischen Zubereitungen nach einem der Ansprüche 1 bis 10, als Hautpflegeprodukt sowie als Aftersun-Produkt.
